## Europäisches Patentamt
## European Patent Office
## Office européen des brevets

(11) Publication number: **0 155 334**
**A1**

# EUROPEAN PATENT APPLICATION

(21) Application number: **84103105.7**

(22) Date of filing: **21.03.84**

(51) Int. Cl.⁴: **C 07 C 103/38**
**//A61K7/06**

(43) Date of publication of application:
**25.09.85 Bulletin 85/39**

(84) Designated Contracting States:
**AT BE CH DE FR GB IT LI LU NL SE**

(71) Applicant: **RICHARDSON VICKS LIMITED**
**Rusham Park Whitehall Lane**
**Egham Surrey TW20 9NW(GB)**

(72) Inventor: **Hoddinott, David Michael**
**37 Park Road**
**Egham Surrey(GB)**

(74) Representative: **Vossius Vossius Tauchner Heunemann Rauh**
**Siebertstrasse 4 P.O. Box 86 07 67**
**D-8000 München 86(DE)**

(54) **Manufacture of pantyl/d-panthenol mixtures.**

(57) A method of producing mixtures of pantyl/d-panthenol mixtures directly is provided by reacting d-pantolactone with a mixture of 3-ethoxy propylamine and propanolamine whereby the d-pantolactone reacts simultaneously with each amine in the same reaction vessel and produces pantyl and d-panthenol in the same weight % ratio as the ratio of gram molecules of 3-ethoxy propylamine to gram molecules of propanolamine.

Our Ref.: S 941 EP
Case: RVL-1283 EP
Richardson-Vicks Limited
Egham, Surrey, GB

March 24, 1984

# MANUFACTURE OF PANTYL/d-PANTHENOL MIXTURES

## Field of the Invention

This invention relates to a reaction mechanism for producing mixtures of pantyl and d-panthenol in a single reaction sequence.

## Background of the Invention

It has been previously known that d-panthenol, namely (R)2,4-dihydroxy-N-(3-hydroxypropyl)-3,3-dimethylbutamide, and pantyl, the ethyl ether of d-panthenol, are used as conditioning agents in shampoos and the like. Moreover, it has been known to utilize a mixture of 3 parts pantyl and 7 parts d-panthenol as such conditioning agents. Even more recently it has been discovered that mixtures of 9 parts pantyl and 1 part d-panthenol impart even more desirable conditioning properties to shampoos, hair lotions and conditioners containing such mixtures of pantyl and d-panthenol.

Heretofore it has been necessary to produce pantyl and d-panthenol separately and then mix the two products to obtain the desired mixtures. It would be highly desirable if such mixtures of pantyl and d-panthenol for use in shampoos and other hair preparations could be manufactured in a simple and simultaneous process.

## Summary of the Invention

A method of producing mixtures of pantyl/d-panthenol mixtures directly is provided by reacting d-pantolactone with a mixture of 3-ethoxy propylamine and propanolamine whereby the d-pantolactone reacts simultaneously with each amine in the same reaction vessel and produces pantyl and d-panthenol in the same weight % ratio as the ratio of gram molecules of 3-ethoxy propylamine to gram molecules of propanolamine.

## Detailed Description of the Invention

In the process of this invention a mixture of 3-ethoxy propylamine and propanolamine is reacted with d-pantolactone over a period of up to about 3 to 4 hours at a temperature of about 80°C or less after which the reaction product is cooled to about 60°C or less and packaged. The resulting product is a mixture of pantyl/d-panthenol in the same weight ratio as the ratio of gram molecules of 3-ethoxy propylamine to gram molecules of propanolamine.

The process of this invention is illustrated by but not limited by the following example for the preparation of a mixture of a 9:1 wt ratio of a pantyl/d-panthenol.

### Example

About 12.123 kg 3-ethoxy propylamine (116.9 gram molecules) and about 0.976 kg propanolamine (12.99 gram molecules) were mixed in a reaction vessel and to this mixture of amines about 16.9 kg d-pantolactone (129.9 gram molecules) was steadily added over a period of about 30 minutes at a maximum temperature of about 56°C. After completion of the lactone addition, the reaction mixture was then heated to about 80°C for about 2 hours and the reaction product cooled to about 60°C and stored in a container. Theoretical yield 29.99 kg; observed yield 29.71 kg. The reaction is able to be conducted with no nitrogen blanket and using un-redistilled amines. Analysis of the reaction product using HPLC assay after fourteen days gave the following composition: pantyl 90.36% w/w and d-panthenol 9.63% w/w.

## Claims

1. A method for the direct production of mixtures of pantyl with d-panthenol in a given weight ratio comprising reacting d-pantolactone with a mixture of 3-ethoxy propylamine and propanolamine present in the mixture in a ratio of gram molecules of 3-ethoxy propylamine to gram molecules of propanolamine equivalent to the ratio of pantyl to d-panthenol desired in the product mixture.

2. The method of claim 1 wherein the ratio is about 9 parts weight pantyl to about 1 part by weight d-panthenol.

3. The method of claim 2 wherein the reaction is conducted at a temperature of about 80°C or less for a period up to about 4 hours.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FR-M- 1 711 (HOFFMANN-LA ROCHE) * abstract * | 1-3 | C 07 C 103/38 // A 61 K 7/06 |

-----

**TECHNICAL FIELDS SEARCHED (Int. Cl.4)**

C 07 C 103/00

The present search report has been drawn up for all claims

| Place of search | Date of completion of the search | Examiner |
|---|---|---|
| THE HAGUE | 19-11-1984 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82

BAD ORIGINAL